# FASCICULE DE BREVET EUROPEEN

(11) **EP 4 388 997 B1**
(45) Date de publication et mention de la délivrance du brevet: **30.09.2026**
(21) Numéro de dépôt: 24170379.2
(22) Date de dépôt: 07.06.2021
(51) Int. Cl.: A61B 5/11

(54) **PROCÉDÉ DE TRAITEMENT DE MESURES EFFECTUÉES PAR UN CAPTEUR PORTÉ PAR UNE PERSONNE**
VERFAHREN ZUR VERARBEITUNG VON MESSUNGEN, DIE VON EINEM VON EINER PERSON GETRAGENEN SENSOR AUSGEFÜHRT WERDEN
METHOD FOR PROCESSING MEASUREMENTS PERFORMED BY A SENSOR WORN BY A PERSON

(30) Priorité: 08.06.2020 FR 2005976
(43) Date de publication de la demande: 26.06.2024
(62) Numéro(s) de document de la (des) demande(s) initiale(s) en application de l'article 76 CBE: 21730586.1 / 4 161 358
(73) Titulaire: Panoramic Digital Health, 38380 Saint-Pierre-de-Chartreuse (FR)
(72) Inventeur: HILL, Derek, 38380 Saint-Pierre-de-Chartreuse (FR); TONIN, Luke, Oxford, OX14XR (GB)
(74) Mandataire: INNOV-GROUP

(56) Documents cités:
- EP-A1- 3 474 287
- WO-A1-2018/184829
- JP-B2- 4 928 849
- US-A1- 2005 043 598
- US-A1- 2012 191 397
- US-A1- 2019 339 224
- US-A1- 2020 093 377

## Description

### DOMAINE TECHNIQUE

Le domaine technique de l'invention est le traitement de mesures effectuées par un capteur, en particulier un capteur nomade, porté par un utilisateur. Les mesures peuvent être des mesures de caractéristiques physiologiques ou des mesures du mouvement de l'utilisateur.

### ART ANTERIEUR

Les dispositifs connectés, de type smartphone, montres connectées, patch ou pansements intelligents, comportent des capteurs permettant de mesurer des caractéristiques de leur utilisateur.

De tels dispositifs comportent par exemple des capteurs d'actimétrie permettant d'obtenir des caractéristiques précises relatives au mouvement de l'utilisateur. Ces capteurs peuvent notamment être des accéléromètres, des gyromètres, des magnétomètres, pression. Ces capteurs peuvent être regroupés pour constituer des centrales inertielles. Une application, relativement simple, et très répandue, est par exemple le comptage des pas. Certains dispositifs connectés permettent de réaliser des mesures de caractéristiques physiologiques, par exemple la fréquence cardiaque, la température du corps. Des capteurs ont également été développés pour estimer des niveaux d'oxygénation du sang ou effectuer des analyses de fluides corporels, par exemple la sueur. En étant intégrés dans des smartphones, des montres, ou les « smart devices » (dispositifs intelligents) ces capteurs bénéficient d'un environnement logiciel puissant, comportant des capacités de calculs élevées couplées à des moyens de communication sans fil.

Les documents EP3474287, US20180078219 et WO2016204905 décrivent des capteurs, destinés à être portés par un utilisateur, et mesurant des caractéristiques physiologiques de l'utilisateur. Les mesures résultant des capteurs font l'objet de traitements, de façon à effectuer des corrections, ou d'éliminer des valeurs aberrantes, par exemple des outliers ou des mesures influencées par un mouvement de l'utilisateur.

Les caractéristiques mesurées peuvent être interprétées par des applications d'interprétation mises en œuvre par le dispositif, ou installées sur des serveurs distants. Il peut par exemple s'agir d'applications d'interprétation relatives au bien-être, au coaching sportif, au suivi de l'état de santé de l'utilisateur, ou à la détection de la survenue d'une situation à risque pour ce dernier. D'une façon générale, ce type d'application permet de classifier un état de l'utilisateur : cet état concerne son activité physique, son état de santé, ou un état susceptible d'entraîner un risque. De telles applications sont en constant développement. Citons par exemple les applications permettant de détecter la survenue d'une somnolence chez conducteur, ou l'apparition d'une crise d'épilepsie, ou encore l'apparition de symptômes de pathologies, comme par exemple la maladie de Parkinson, ou l'apnée du sommeil, ou encore la détection d'une chute ou d'un malaise. Citons également les innombrables applications dédiées au suivi de l'activité physique d'un utilisateur.

US20050043598 décrit un capteur implanté, pouvant être calibré pour indiquer une valeur d'une concentration de glucose dans le sang à partir d'une acquisition d'un courant électrochimique résultant de l'oxydation du glucose.

Chaque capteur est piloté par un logiciel de paramétrage dédié, usuellement désigné logiciel embarqué ou "firmware". Ce type de logiciel permet un paramétrage du capteur. Au cours du temps, les logiciels de paramétrage dédiés peuvent faire l'objet de mises à jour, ou de changements. Par conséquent, pour un même type de capteur, le signal généré peut évoluer en fonction de la version du logiciel pilotant le capteur. Or, l'application d'interprétation, à laquelle les signaux sont destinés, ne prend pas nécessairement en compte les évolutions apportées au logiciel de pilotage. Il en résulte un risque quant à la fiabilité des données issues de l'application. En effet, un signal, correspondant à une mesure d'une même grandeur physique, peut être modifié suite à la mise à jour du logiciel de paramétrage du capteur.

Un problème similaire se pose lorsque deux capteurs identiques sont alimentés respectivement avec deux alimentations différentes. Une variation de l'alimentation peut entraîner une différence entre les signaux générés par les capteurs, fussent-ils identiques.

Le même problème se pose lorsqu'une même application reçoit des mesures réalisées par des capteurs différents, mesurant la même caractéristique physique, par exemple deux accéléromètres différents.

Ainsi, les mesures adressées à une même application subissent une variabilité dépendant du capteur utilisé, et de l'environnement du capteur : alimentation, version du logiciel de paramétrage. Les données d'entrée, transmises à une application d'interprétation, et correspondant à une même grandeur physique, peuvent donc varier. Une variabilité des données d'entrée ne peut entraîner qu'une variabilité de l'interprétation fournie par l'application d'interprétation, avec des conséquences sur la fiabilité des résultats fournis par cette dernière. On comprend que cette question est particulièrement importante lorsque l'application d'interprétation concerne l'état de santé du patient, ou la détection de la survenue d'une situation à risque. Pour de telles applications, il est nécessaire de limiter autant que possible la survenue de faux positifs (un état pathologique ou à risque est détecté à tort) ou de faux négatifs (un état pathologique ou à risque n'est pas détecté alors qu'il devrait l'être).

Les inventeurs proposent un procédé permettant d'adresser ce problème. L'objectif est d'aboutir à une meilleure maîtrise des signaux mesurés par des capteurs et transmis à des applications d'interprétation, telles que précédemment décrites.

### EXPOSE DE L'INVENTION

Un premier objet de l'invention est un procédé de traitement de mesures selon la revendication 1.

Selon un mode de réalisation, le procédé comporte, avant l'acquisition des mesures et/ou durant l'acquisition des mesures :
- une vérification de la conformité des valeurs de chaque paramètre du capteur vis-à-vis de valeurs spécifiées, respectivement pour chaque paramètre du capteur, une vérification étant considérée comme
   ∘ négative lorsqu'au moins une valeur d'un paramètre du capteur n'est pas conforme à la valeur spécifiée pour ledit paramètre du capteur;
   ∘ positive lorsque la valeur de chaque paramètre du capteur est conforme à la valeur spécifiée pour ledit paramètre du capteur;
- suite à une vérification négative, une mise à jour de chaque valeur de paramètre du capteur non conforme, en remplaçant chaque valeur de paramètre non conforme par une valeur spécifiée pour ledit paramètre.

Le procédé peut comporter, au cours de l'acquisition des mesures, plusieurs vérifications successives de la conformité des valeurs de chaque paramètre, le procédé étant tel que suite à une vérification négative, les mesures acquises depuis une précédente vérification positive et/ou jusqu'à une vérification positive suivante sont considérées comme douteuses ou non valides.

Le procédé peut comporter :
- une prise en compte d'une séquence de mesures type par le dispositif connecté ;
- une transmission de la séquence de mesures type à l'application d'interprétation, la transmission étant effectuée par le dispositif connecté ;
- une comparaison de la séquence de mesure transmise à l'application d'interprétation avec la séquence de mesure type.

Chaque capteur mesure une grandeur physique ou chimique. Le procédé fait en sorte que deux mesures, correspondant à une même grandeur, et respectivement acquises par deux capteurs de mesure différents, aboutissent, suite au traitement par le modèle de calibration, à une même mesure de référence. Une mesure de référence peut correspondre à une mesure qui serait obtenue par un capteur de référence.

Lors de l'étape i), le capteur de mesure peut être disposé sur un fantôme, représentatif d'au moins un état de l'utilisateur, les données de référence étant obtenues à partir du fantôme. Le fantôme peut comporter un capteur de référence, le capteur de référence délivrant de mesures de référence à chaque instant de calibration.

Lors de l'étape i), le capteur de calibration peut être disposé sur au moins un individu test, l'individu test portant également un capteur de référence, le capteur de référence délivrant une mesure de référence à chaque instant de calibration.

De préférence, le modèle de calibration met en œuvre un algorithme d'intelligence artificielle supervisé, l'algorithme d'intelligence artificielle supervisé étant paramétré au cours de la phase de calibration. L'algorithme d'intelligence artificielle supervisé peut comporter un réseau de neurones. Le réseau de neurones peut être un réseau de neurones récurrent, et de préférence bidirectionnel, et comporter une couche d'entrée et une couche de sortie, de telle sorte qu'au cours du traitement de chaque mesure par le modèle de calibration :
- chaque mesure acquise par le capteur de mesure forme une couche d'entrée du réseau de neurones ;
- la couche de sortie correspond à l'estimation d'au moins une mesure de référence.

Le procédé peut comporter, entre les étapes i) et ii), une synchronisation temporelle des mesures de calibration par rapport aux mesures de référence.

Le capteur peut comporter au moins :
- un capteur de mouvement, de type accéléromètre et/ou gyromètre et/ou magnétomètre;
- et/ou un capteur de pression ;
- et/ou un capteur optique ;
- et/ou un capteur chimique ;
- et/ou un capteur électrique ;
- un capteur de température ;
- et/ou un capteur physiologique, configuré pour mesuré une caractéristique physiologique de l'utilisateur.

La caractéristique physiologique peut être choisie parmi :
- une température corporelle ;
- une pression sanguine ;
- un taux d'oxygène ou de dioxyde de carbone dans le sang ;
- une caractéristique de l'activité respiratoire, par exemple une fréquence respiratoire ;
- une caractéristique de l'activité cardiaque, par exemple une fréquence cardiaque ;
- un niveau de sudation ;
- une caractéristique d'une activité musculaire ;
- une caractéristique d'une activité neuronale.

L'état de l'utilisateur peut être choisi parmi :
- un état décrivant une activité physique de l'utilisateur ;
- un état de stress ;
- un état de sommeil ou de somnolence ;
- un état pathologique ;
- un état symptomatique
- un état correspondant à une survenue d'une situation à risque pour l'utilisateur.

L'utilisateur peut être un être humain vivant ou un animal vivant.

L'application d'interprétation peut être mise en œuvre par un microprocesseur intégré dans le dispositif connecté, ou par un microprocesseur distant du dispositif connecté, et relié à ce dernier par une liaison filaire ou sans fil.

Un deuxième objet de l'invention est un dispositif connecté selon la revendication 13. Selon un mode de réalisation, le dispositif connecté est destiné à être porté par un utilisateur, et comporte un capteur de mesure, le capteur de mesure étant configuré pour acquérir, à différents instants de mesure, une mesure représentative d'un mouvement de l'utilisateur ou d'une caractéristique physiologique de l'utilisateur, le capteur de mesure étant paramétré par des paramètres du capteur, chaque paramètre du capteur étant mémorisé dans un registre de commande d'un circuit de commande du capteur, le dispositif étant configuré pour activer une application d'interprétation, l'application d'interprétation étant programmée pour estimer, à partir des mesures acquises par le capteur de mesure, un état de l'utilisateur, l'état de l'utilisateur étant choisi parmi plusieurs états prédéterminés. Le dispositif peut comporter une unité centrale. L'unité centrale peut être programmée pour mettre en œuvre les étapes de vérification de la conformité de la valeur de chaque paramètre et l'éventuelle étape de mise à jour de chaque valeur de paramètre non conforme selon le premier objet de l'invention ;
L'unité centrale peut être programmée pour :
- traiter de chaque mesure par un modèle de calibration, tel que décrit en lien avec le premier objet de l'invention, de façon à obtenir, à partir de chaque mesure, une mesure de référence ;
- transmettre chaque mesure de référence à l'application d'interprétation.

Les opérations réalisées par l'unité centrale, relatives à la conformité des paramètres et/ou à l'application du modèle de calibration, peuvent être pilotées par une application d'interface. L'application d'interface constitue une interface entre le capteur et l'application d'interprétation.

L'invention sera mieux comprise à la lecture de l'exposé des exemples de réalisation présentés, dans la suite de la description, en lien avec les figures listées ci-dessous.

### FIGURES

La figure 1 représente des séquences mesures acquises simultanément par deux capteurs différents ;
La figure 2 schématise un exemple de dispositif permettant une mise en œuvre de l'invention.
La figure 3A montre les principales étapes d'une première composante d'un procédé selon l'invention.
La figure 3B montre un processus de vérification périodique d'un registre d'un capteur de mesure.
La figure 4A montre les principales étapes d'une deuxième composante d'un procédé selon l'invention.
La figure 4B illustre une détermination d'une fonction de synchronisation.
Les figures 4C, 4D et 4E schématisent respectivement un premier réseau de neurones récurrent, un deuxième réseau de neurones récurrent et un troisième réseau de neurones.
La figure 4F résume une structure d'un modèle de calibration préféré.
La figure 5A représente deux séquences de mesure expérimentales acquises à partir de deux capteurs de même type, paramétrés différemment.
La figure 5B montre les séquences de mesure représentées sur la figure 5A, ainsi qu'une séquence de mesure ayant fait l'objet de l'application d'un modèle de calibration, de façon à obtenir une estimation de mesures de référence.
La figure 5C est un détail de la figure 5B, entre les instants t=300 et t=400 de cette dernière.
La figure 6A représente deux séquences de mesure expérimentales acquises à partir de deux capteurs différents mesurant une même grandeur physique, en l'occurrence une accélération.
La figure 6B montre les séquences de mesure représentées sur la figure 6A, ainsi qu'une séquence de mesure ayant fait l'objet de l'application d'un modèle de calibration, de façon à obtenir une estimation de mesures de référence.
La figure 7A représente deux séquences de mesure expérimentales acquises à partir de deux capteurs différents mesurant une même grandeur physique, en l'occurrence une accélération.
La figure 7B montre les séquences de mesure représentées sur la figure 7A, ainsi qu'une séquence de mesure ayant fait l'objet de l'application d'un modèle de calibration, de façon à obtenir une estimation de mesures de référence.

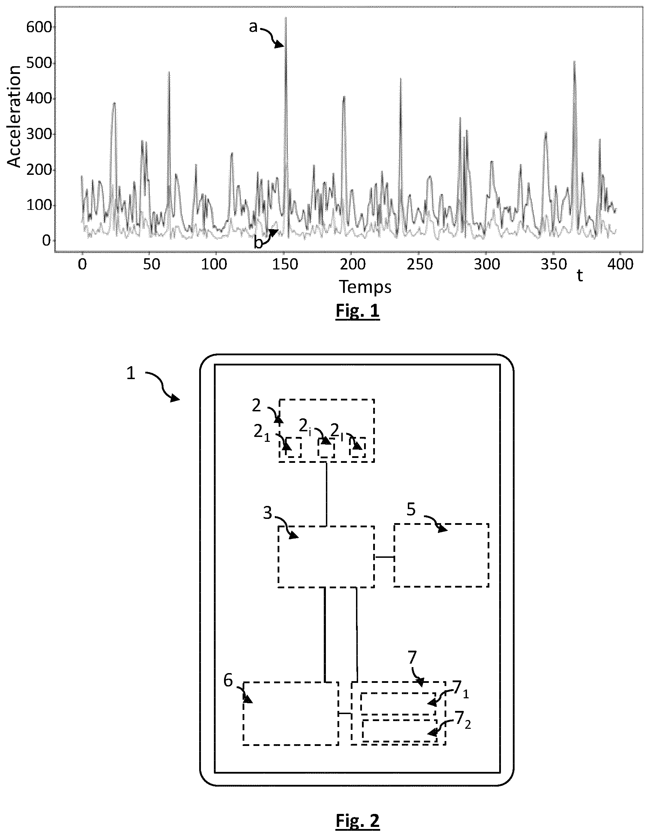

   Sur les figures 1, 3B, 5A à 5C, 6A, 6B, 7A et 7B, l'axe des ordonnées correspond aux mesures et l'axe des abscisses correspond au temps.

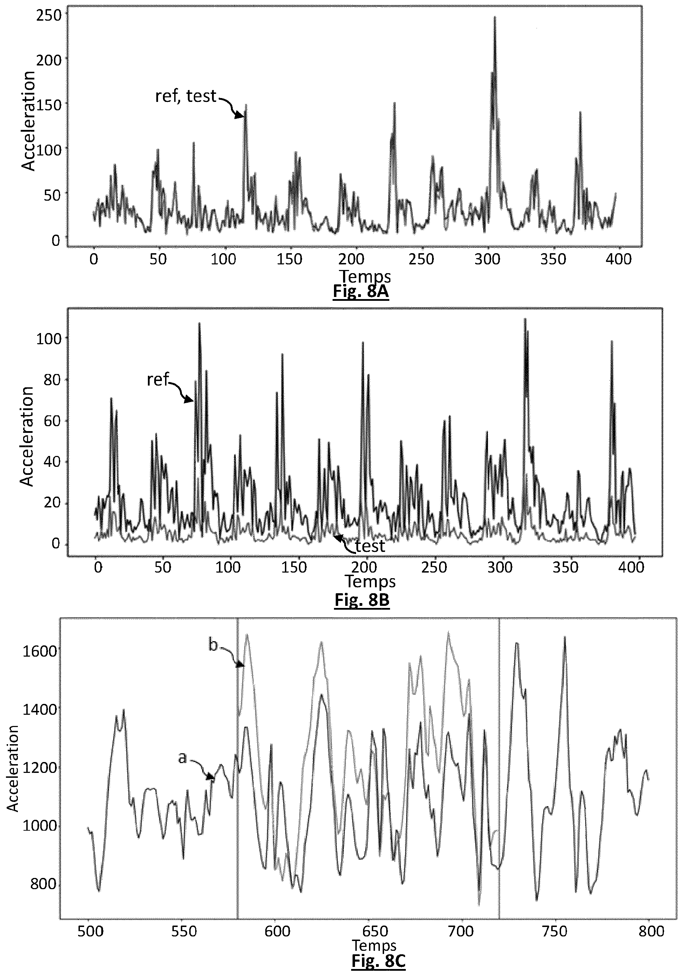

La figure 8A montre des mesures résultant d'un capteur de référence et d'un capteur de test, ayant été paramétrés selon une même configuration de référence.
La figure 8B montre des mesures résultant du capteur de référence et du capteur de test décrits en lien avec la figure 8A.
La figure 8C montre l'effet d'un mauvais paramétrage temporaire (courbe b) par rapport à une configuration de référence (courbe a). La figure 8C montre également une détection du mauvais réglage, ainsi que l'effet de la correction induite par l'invention.

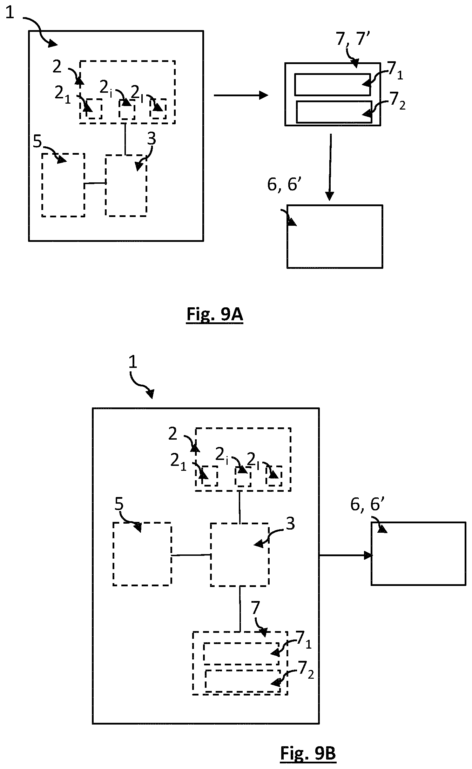

Les figures 9A et 9B illustrent des variantes de dispositif permettant une mise en œuvre de l'invention.

### EXPOSE DE MODES DE REALISATION PARTICULIERS

Sur la figure 1, on a représenté des mesures d'accélération, acquises à différents instants, acquises par deux capteurs de mouvement disposés sur le même membre d'une personne. Les mesures ont été obtenues en utilisant deux accéléromètres identiques, implantés sur deux centrales inertielles différentes, ces dernières étant respectivement pilotées un circuit de prétraitement mettant en œuvre un filtre passe-bas.

Plus précisément, les deux accéléromètres ont été utilisés en mettant en œuvre deux fréquences de coupure différentes du filtre passe-bas. Il en résulte deux courbes (courbe a et courbe b). Chaque pic d'accélération est correctement détecté. Cependant, il apparaît une différence significative au niveau de l'amplitude des signaux détectés. De plus, les inventeurs ont observé que le ratio entre les amplitudes ne se résume pas à un simple facteur multiplicatif.

L'invention décrite ci-après permet d'adresser ce type de situation. Dans l'exemple décrit par la suite, on se réfère, de façon non limitative, à un dispositif connecté 1 prenant la forme d'un téléphone portable connecté de type smartphone. Le dispositif connecté 1 est destiné à être porté par un utilisateur, ce dernier étant le plus souvent un être humain ou animal vivant. Le dispositif 1 est notamment destiné à être disposé contre le corps de l'utilisateur, dans la poche d'un vêtement ou en étant appliqué directement au contact du corps de l'utilisateur. Le dispositif connecté 1 est configuré pour être relié à un réseau de communication sans fil, par exemple, et à titre non limitatif, un réseau de type wifi ou 4G ou 5G ou bluetooth.

L'invention s'applique à d'autres types de dispositifs connectés, par exemple une montre, ou une tablette ou un pansement intelligent ou un patch. Le dispositif 1, représenté sur la figure 2, comporte un capteur de mesure 2, ce dernier étant paramétré par différents registres de commande 2₁, 2ᵢ...2ₗ. Les registres sont intégrés un circuit électronique de commande faisant partie du capteur. Le circuit électronique de commande peut comporter un microprocesseur de commande auquel cas les registres sont intégrés dans une mémoire reliée au microprocesseur. La fonction du circuit électronique de commande est de piloter l'acquisition de mesures par le capteur et/ou un prétraitement de mesures acquises par le capteur. Par prétraitement, on entend un traitement de mesures effectué au niveau du capteur, par le circuit électronique de commande. Le prétraitement est réalisé préalablement à une transmission des mesures à une application d'interface décrite par la suite Le capteur de mesure 2, ainsi que les registres, sont pilotés par un logiciel de paramétrage 3, ou usuellement désigné "firmware". Le dispositif 1 comporte une unité centrale 5, par exemple de type CPU (Central Processing Unit - Unité centrale de traitement) cette dernière effectuant une interface entre les différents composants présents dans le dispositif. L'unité centrale 5 peut notamment contrôler le capteur de mesure 2 par l'intermédiaire du logiciel de paramétrage 3. L'unité centrale 5 peut également être une unité de microcontrôle, usuellement désignée par l'acronyme MCU (Microcontroler Unit)

Le capteur de mesure 2 peut être, ou comporter, un capteur de mouvement, par exemple un accéléromètre, un magnétomètre, un gyromètre. Il peut par exemple s'agir d'une centrale inertielle. Le capteur de mesure 2 peut être ou comprendre un capteur de pression, un capteur optique, un capteur de température, un capteur chimique, un capteur de gaz, un capteur électrique, par exemple une électrode. Il peut également s'agir d'un capteur physiologique, configuré pour déterminer une caractéristique physiologique de l'utilisateur. Par caractéristique physiologique, on entend par exemple, et de façon non limitative :
- une température corporelle ;
- une pression sanguine, par exemple une pression artérielle ;
- une caractéristique de l'activité cardiaque, par exemple la fréquence cardiaque ou un intervalle inter-battements ;
- une caractéristique respiratoire, par exemple une fréquence respiratoire ;
- une caractéristique de l'activité musculaire ;
- une caractéristique de l'activité neuronale, par exemple une activité neuronale mesurée par une électrode ;
- une caractéristique de la sudation, par exemple un niveau de sueur ;
- une concentration d'oxygène ou de dioxyde de carbone dans le sang ;

D'une façon générale, le capteur de mesure 2 est configuré pour mesurer une grandeur physique ou chimique dépendant d'un état dans lequel se trouve l'utilisateur.

Le dispositif 1 met en œuvre une application d'interprétation 6, stockée dans une mémoire. L'application d'interprétation peut être mise en œuvre par l'unité centrale 5, en faisant appel à des mesures effectuées par le capteur de mesure 2. Selon une variante, l'application d'interprétation est stockée sur un serveur distant, relié au dispositif 1 par une liaison, de préférence une liaison sans fil.

D'une façon générale, l'application d'interprétation 6 est alimentée par au moins une séquence de mesures, provenant d'au moins un capteur de mesure. Par séquence de mesures, on entend un ensemble de mesures respectivement acquises à différents instants de mesure, et en général des instants de mesure successifs. Le terme application d'interprétation désigne le fait que l'application prend en compte les mesures établies par le capteur de mesure pour en établir une interprétation. Comme mentionné en lien avec l'art antérieur, il peut s'agir d'une application visant à déterminer un état dans lequel se trouve l'utilisateur, parmi des états prédéterminés. Parmi les états prédéterminés, on peut citer un état de stress, de fatigue, de somnolence, d'activité physique, de vigilance, de repos, de sommeil, ou un état considéré comme pathologique ou potentiellement pathologique, ou un état symptomatique. L'application d'interprétation peut permettre d'établir un bilan de l'activité physique de l'utilisateur durant une période déterminée. Elle peut également permettre de détecter une survenue d'un risque potentiel pour l'utilisateur.

Une application particulièrement envisagée est l'obtention de caractéristiques de l'utilisateur pour assister l'élaboration d'un examen clinique ou la détection de la survenue d'un état pathologique. Aussi, il est important que la fiabilité de l'application d'interprétation 6 soit aussi élevée que possible, en limitant les occurrences de faux positifs ou de faux négatifs, comme indiqué dans l'art antérieur.

Le dispositif 1 comporte une application d'interface 7, stockée dans une mémoire, destinée à traiter les mesures issues du capteur de mesure, avant que ces dernières soient adressées à l'application d'interprétation. L'objectif de l'application d'interface est de remédier aux défauts décrits en lien avec la figure 1 ainsi que dans l'art antérieur. Il s'agit de faire en sorte que des capteurs différents mesurant, au même moment, une même grandeur physique ou chimique, transmettent des mesures comparables à l'application d'interprétation 6. En d'autres termes, il s'agit de réduire la variabilité des données transmises à l'application d'interprétation 6.

L'application d'interface comporte une première composante 7₁, destinée à interagir avec le capteur de mesure 2, par le biais du logiciel de paramétrage 3 associé au capteur. Elle comporte une deuxième composante 7₂ destinée à traiter les mesures issues du capteur, de façon à transmettre des mesures traitées à l'application d'interprétation. Ainsi, l'application d'interface 7 constitue une interface entre le capteur 2 et l'application d'interprétation 6. Elle agit sur le capteur de mesure, de façon à s'assurer que le paramétrage de ce dernier correspond à des spécifications prédéfinies. Elle agit également sur les données mesurées par le capteur de mesure, de façon à assurer une uniformisation de ces dernières par rapport à des mesures de référence.

L'objectif est qu'après le traitement effectué par l'application d'interface 7, les mesures transmises à l'application d'interprétation 6 puissent être considérées comme indépendantes du dispositif connecté 1 porté par l'utilisateur, et plus précisément du capteur de mesure 2. En d'autres termes, l'application d'interface est configurée de telle sorte que deux capteurs différents 2 (par exemple deux accéléromètres), appartenant à deux dispositifs différents 1 (par exemple deux téléphones portables), et soumis à des conditions identiques, transmettent, après traitement par l'application d'interface, des séquences de données identiques, ou considérées comme telles, à l'application d'interprétation.

L'application d'interface 7 permet également de se prémunir de variations affectant les mesures acquises par le capteur suite à :
- une modification du protocole de communication entre le capteur 2 et l'application d'interprétation 6;
- un changement d'alimentation du dispositif 1 comportant le capteur 2;
- une mise à jour du logiciel de paramétrage du capteur, ou toute autre application téléchargée dans le dispositif 1, et pouvant affecter les mesures effectuées par le capteur 2.

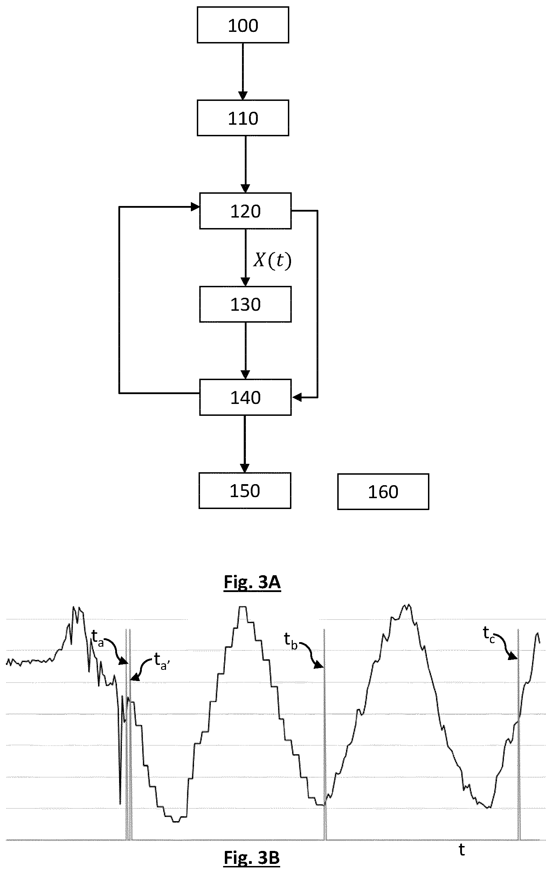

La figure 3A schématise les principales étapes d'un procédé mis en œuvre par la première composante 7₁.

### Etape 100 : Prise en compte de paramètres du capteur

Au cours de cette étape, on prend en compte des paramètres du capteur. Les paramètres du capteur sont stockés dans un ou plusieurs registres de commande 2ᵢ du capteur 2. Comme précédemment décrit, les registres sont formés 2ᵢ dans un circuit électronique de commande faisant partie du capteur 2. Les paramètres du capteur peuvent comprendre des paramètres d'acquisition en fonction desquels le capteur 2 effectue chaque mesure. Il s'agit de paramètres d'acquisition régissant le fonctionnement du capteur 2, disponibles dans une spécification. Les paramètres d'acquisition peuvent notamment dépendre de la finalité dans laquelle les mesures sont effectuées. Il est supposé qu'on dispose d'une spécification, mentionnant les paramètres d'acquisition d'un capteur. Un paramètre d'acquisition peut être choisi parmi :
- la fréquence de mesure (fréquence d'échantillonnage) ;
- un paramètre d'ajustement d'un filtre fréquentiel appliqué à chaque mesure, par exemple une fréquence de coupure d'un filtre passe-bas ou d'un filtre passe-haut ;
- une durée de chaque séquence de mesures ;
- un intervalle temporel entre deux séquences de mesures successives ;

Le capteur 2 peut effectuer une étape de prétraitement des mesures, auquel cas un paramètre du capteur peut être un paramètres de prétraitement régissant un prétraitement. Un tel prétraitement peut être réalisé au niveau du capteur 2 (par exemple par le circuit électronique de commande) ou du logiciel de paramétrage 3 associé au capteur. Un paramètre de prétraitement peut comporter :
- un paramètre représentant un seuil de détection d'un mouvement ;
- un facteur de pondération affecté à une mesure, en particulier lorsqu'une mesure est combinée à d'autres mesures. C'est par exemple le cas lorsqu'une mesure est une composante d'une grandeur mesurée, auquel cas la mesure peut être combinée à d'autres mesures représentant respectivement d'autres composantes.

D'une façon générale, le prétraitement est effectué au niveau du capteur 2, préalablement à une transmission des mesures à l'application d'interface 7.

Les paramètres du capteur peuvent avoir être préalablement spécifiés par l'application d'interprétation. Selon un mode de réalisation, la spécification, comportant les paramètres d'acquisition, est transmise par l'application d'interprétation 6 à l'application d'interface 7.

### Etape 110 : initialisation des registres.

Au cours de cette étape, en utilisant les paramètres du capteur spécifiés lors de l'étape 100, l'application d'interface 7 vérifie et/ou initialise les registres 2ᵢ de chaque capteur 2, de façon que ces registres soient conformes aux paramètres du capteur spécifiés.

Les étapes 120 à 150 sont mises en œuvre à chaque instant de mesure d'une séquence de mesures.

### Etape 120 : réalisation d'une mesure par le capteur de mesure

Au cours de cette étape, le capteur de mesure effectue une mesure *X(t)* à un instant de mesure t, selon les paramètres du capteur spécifiés dans les registres qui lui sont associés. Par mesure, on entend:
- soit des mesures brutes, acquises par le capteur ;
- soit des mesures brutes ayant subi un prétraitement, par exemple un calcul d'une valeur moyenne, par exemple une moyenne glissante, ou un calcul d'une valeur médiane, ou une occurrence de mesures particulières. Une mesure particulière peut correspondre à des mesures brutes successives dont l'occurrence correspond à une situation particulière. Par exemple, lorsque le capteur est un accéléromètre, une mesure particulière peut être une double tape exercée par l'utilisateur sur le dispositif. Le prétraitement, effectué au niveau du capteur, peut détecter l'occurrence d'une double tape et transmettre le fait que cette occurrence est apparue. Cela peut correspondre à une situation dans laquelle l'utilisateur souhaite commencer une nouvelle séquence de mesures. Le prétraitement peut également permettre de fournir une mesure établie à partir des mesures brutes. Il peut s'agir par exemple d'un comptage de pas lorsque le capteur est un accéléromètre.

### Etape 130 : Vérification des registres

Au cours de cette étape, l'application d'interface 7 vérifie que le contenu de chaque registre 2ᵢ n'a pas été modifié. Il s'agit de s'assurer que les registres 2ᵢ associés au capteur 2 n'ont pas été modifiés. Il n'est pas nécessaire que l'étape 130 soit effectuée à chaque instant de mesure. Elle peut être mise en œuvre périodiquement, entre un nombre prédéterminé d'instants de mesure, par exemple toutes les 5 minutes. Lorsque les registres n'ont pas été modifiés, et sont conformes au paramétrage défini lors de l'étape 110, les mesures effectuées depuis la vérification précédente sont considérées comme valides. Lorsque l'étape 130 détecte une modification d'un registre :
- les mesures effectuées depuis la vérification conforme précédente et/ou jusqu'à la vérification conforme suivante sont considérées comme non valides, ou douteuses (cf. étape 150).
- les registres présentant une non conformité sont mis à jour de façon à être conformes aux paramètres spécifiés.

Etape 140 : incrémentation de l'instant de mesure et retour à l'étape 120 .

### Etape 150 : Validation ou invalidation de mesures

Les mesures *X(t)* effectuées entre deux vérifications consécutives, et considérées comme conformes sont associées à un indicateur (ou label) de validité.

Lorsqu'une vérification est non conforme, les mesures effectuées :
- depuis la vérification conforme précédente ,
- et/ou jusqu'à la vérification conforme suivante ,
sont considérées comme invalides ou douteuses, auquel cas elles peuvent être associées à un indicateur d'invalidité. Une mesure considérée comme douteuse peut faire l'objet d'une correction post acquisition.

Cela permet d'éviter que les mesures considérées comme invalides ou douteuses soient prises en compte, par la suite, par l'application d'interprétation 6.

La figure 3B illustre le processus de vérification des mesures décrit en lien avec l'étape 130. Sur cette figure, on a représenté des mesures X(t) effectuées au cours du temps, décrivant une pseudo-sinusoïde. L'axe des abscisses correspond au temps t et l'axe des ordonnées correspond aux mesures *X*(*t*) effectuées par le capteur de mesure 2. Une vérification des registres, telle que décrite dans l'étape 130, est effectuée de façon périodique. Sur la figure 3B, chaque vérification est matérialisée par un trait vertical. On a représenté trois vérifications successives, effectuées aux instants tₐ, t_{b} et t_{c}. La vérification effectuée à l'instant tₐ détecte une modification d'un des registres. Une correction de chaque registre est effectuée à l'instant t_{a'} postérieur à l'instant tₐ. Les vérifications de la conformité des registres effectuées aux l'instants t_{b} et t_{c} ne détecte pas d'anomalie. Les mesures effectuées entre les instants tₐ et t_{b} sont considérées comme non valides, parce que la vérification effectuée à l'instant tₐ a détecté une anomalie. De ce fait, les mesures effectuées entre les instants tₐ et t_{b} sont affectées d'un indicateur de non validité. Les mesures effectuées entre les instants t_{b} et t_{c} sont valides.

Selon un mode de réalisation optionnel, le procédé comporte une étape 160 de prise en compte d'une séquence de mesures type. Une telle séquence peut être téléchargée sur une base de données, publique ou liée à l'application d'interprétation 6. Elle forme alors une séquence type originale. Au cours de l'étape 160, l'application d'interface 7 transmet la séquence de mesures type à l'application d'interprétation 6. On compare ensuite la séquence type originale avec la séquence reçue par l'application d'interprétation 6. La comparaison peut comporter une détermination d'un indicateur de qualité de la séquence type transmise. L'indicateur de qualité peut être une erreur quadratique entre la séquence type originale et la séquence type transmise. Lorsque l'indicateur de qualité franchit un seuil prédéterminé, la transmission est considérée comme corrompue. Un avertissement est alors transmis à l'application d'interprétation 6, mentionnant le fait que les données qui lui sont transmises sont potentiellement corrompues. Dans le cas contraire, la transmission est jugée correcte. Cette étape permet de vérifier la qualité du protocole de transmission entre les données traitées par l'application d'interprétation 6 et l'application d'interface 7.

On va à présent décrire les principales étapes de la deuxième composante 7₂ de l'application d'interface. Au cours de cette étape, on effectue une transformation des mesures délivrées par le capteur 2, de telle sorte qu'après transformation, les mesures correspondent à des mesures qui auraient été acquises à l'aide d'un capteur de référence. Cette étape suppose l'établissement d'un modèle de calibration. Le modèle de calibration est paramétré au cours d'une phase de calibration, préalablement effectuée.

L'objectif de la phase de calibration est d'établir les paramètres du modèle de calibration, de telle sorte que le modèle de calibration soit ensuite appliqué à chaque séquence de mesures résultant du capteur 2. La phase de calibration comporte plusieurs instants de calibration t', formant une ou plusieurs séquences de calibration. L'établissement du modèle de calibration suppose le recours à des mesures de référence. Trois possibilités peuvent être envisagées :
- Les mesures de référence sont effectuées en disposant un capteur, du même type que le capteur de mesure équipant le dispositif 1, sur un fantôme configuré pour faire varier la grandeur physique. Durant la séquence de calibration, la grandeur physique évolue dans une plage de mesure cohérente avec des valeurs susceptibles d'être mesurées dans des conditions réelles. Cette possibilité convient particulièrement lorsque la grandeur physique mesurée est un mouvement. Dans ce cas, les valeurs de référence peuvent être obtenues en disposant le capteur de mesure sur un bras robotisé, faisant office de fantôme, et dont on maîtrise le mouvement. Le bras robotisé peut par exemple mimer le mouvement d'un avant-bras, ou d'une jambe, ou d'une cuisse. Les valeurs de référence sont établies en fonction des mouvements programmés du fantôme. Elles peuvent résulter de la programmation des mouvements du fantôme. On peut également utiliser un fantôme dont on maîtrise la température, ou les propriétés optiques ou chimiques, et dont on peut programmer une évolution temporelle de la température ou des propriétés optiques ou chimiques.
- Les mesures de référence sont effectuées en disposant un capteur de référence, du même type que le capteur de mesure 2 équipant le dispositif 1, sur un fantôme tel que décrit dans l'alinéa précédent. Un capteur de référence, considéré comme fournissant des mesures exactes, est également disposé sur le fantôme.
- Les mesures de référence sont effectuées en équipant une population d'individus de test d'un capteur de référence, considéré comme fournissant des mesures exactes. Chaque individu test de la population est également équipé d'un capteur de mesure 2. La population comporte au moins un individu, et de préférence plusieurs individus test différents. Il est préférable que la population comprenne des individus test considérés comme représentatifs de l'application d'interprétation 6 utilisée. Par exemple, lorsque l'application d'interprétation vise à déterminer un état d'un utilisateur, il est préférable qu'au cours de la calibration, au moins un individu test de la population soit dans un état ciblé par l'application (ce qui correspond à un vrai positif) et qu'au moins un individu test ne soit pas dans un état ciblé par l'application (ce qui correspond à un vrai négatif).

Plusieurs séquences de calibration peuvent être réalisées pour chaque individu test.

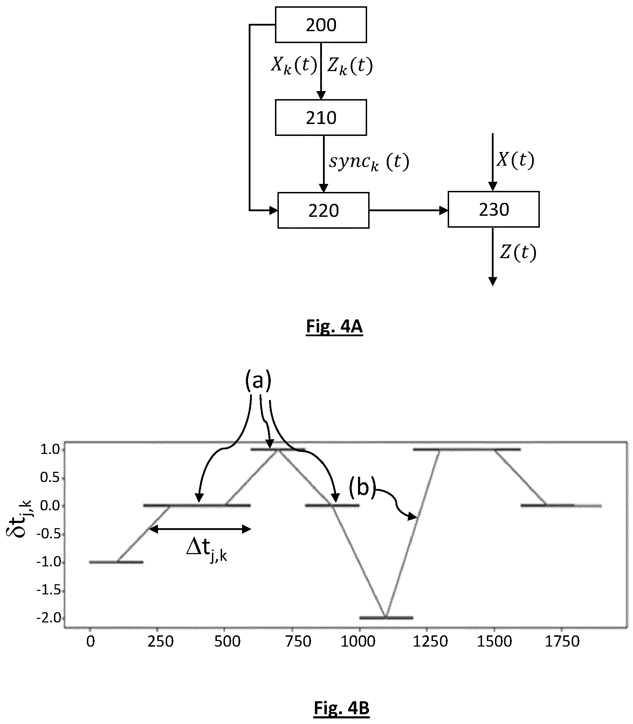

La phase de calibration comporte les étapes 200 à 230 décrites sur la figure 4A.

Etape 200 : acquisition des mesures de calibration *Xₖ*(*t*) et des mesures de référence *Zₖ*(*t*) Au cours de différents instants de calibration t, on acquiert :
- des mesures de calibration *Xₖ*(*t*), données par le capteur de mesure 2, ou par chaque capteur de mesure 2 utilisé lors de la calibration ;
- des mesures de référence *Zₖ*(*t*) données, selon les cas, par l'éventuel capteur de référence ou par les paramètres de fonctionnement du fantôme

Les mesures de calibration *Xₖ*(*t*) correspondent aux mesures acquises avec le capteur de mesure 2 durant la phase de calibration. Les mesures de calibration *Xₖ*(*t*) forment différentes séquences de calibration, l'indice *k* indexant chaque séquence de calibration.

Etape 210 : synchronisation temporelle des mesures de calibration et des mesures de référence.

Au cours de cette étape optionnelle, les mesures acquises par le capteur de mesure et les mesures de référence sont recalées dans le temps. Il s'agit de prendre en compte une éventuelle dérive temporelle affectant les horloges commandant respectivement le capteur de mesure ainsi que l'éventuel capteur de référence. L'objectif de cette phase est d'obtenir à une synchronisation la plus précise possible entre les mesures de référence et les mesures de calibration.

L'étape 210 peut comprendre l'établissement d'une fonction de synchronisation. La fonction de synchronisation peut être obtenue en divisant chaque séquence de calibration *k* en *J* segments temporels Δt_{j,k} de courte durée, par exemple une durée comprise entre 1s et 5s, et par exemple d'une durée égale à 2 s. *J* est un entier désignant le nombre de segments temporels divisant une même séquence de calibration.

Sur chaque segment temporel Δt_{j,k}, on détermine un coefficient de corrélation entre les mesures de références et les mesures de calibration, en prenant en compte différents décalages temporels δt entre les mesures de référence et les mesures de calibration, y compris un décalage temporel nul. Les décalages temporels considérés sont de préférence faibles. Il peut s'agir de décalages temporels δt d'étendant de part et d'autre de 0. Par exemple δt = 0 ; δt = ±10 ms ; δt = ±20 ms ; δt = ±30 ms ...On identifie le décalage temporel δt_{j,k} pour lequel la corrélation est maximale. A chaque segment temporel Δt_{j,k} d'une séquence de calibration *k* correspond alors le décalage temporel δt_{j,k} ainsi identifié. Une fonction de synchronisation *syncₖ* est établie pour chaque séquence de calibration k, à partir des segments temporel Δt_{j,k} établis, de telle sorte que : ${sync}_{k} \left(t∈Δ{t}_{j , k}\right) = t + {δt}_{j , k}$

La fonction de synchronisation *syncₖ* est ensuite appliquée soit aux mesures de référence, soit au mesures de calibration, de façon à obtenir une synchronisation temporelle précise. Si *Zₖ*(*t*) et *Xₖ*(*t*) sont respectivement les mesures de référence et de calibration acquises au cours d'une même séquence de calibration *k,* la prise en compte de la fonction de synchronisation *syncₖ* prend le forme d'une composition, de telle sorte que : ${Z}_{k} \left(t\right) \to {Z}_{k} \left(t\right) ∘ {sync}_{k} \left(t\right)$ Ou ${X}_{k} \left(t\right) \to {X}_{k} \left(t\right) ∘ {sync}_{k} \left(t\right)$

La figure 4B représente un exemple d'évolution temporelle de fonction de synchronisation (courbe (a)). On voit que selon l'expression (1), la fonction de synchronisation *syncₖ* est discontinue. Sa valeur change brusquement entre chaque segment temporel *δt_{j,k}.* Afin d'éviter de telles discontinuités, l'étape 210 peut comprendre une interpolation de la fonction de synchronisation telle qu'explicitée dans (1), de façon à obtenir une fonction de synchronisation continue. L'interpolation est par exemple linéaire ou polynomiale. On a représenté, sur la figure 4B, une fonction de synchronisation interpolée. Elle correspond à la courbe (b).

### Etape 220 : Détermination du modèle de calibration.

Suite à l'étape 210, ou suite à l'étape 200, si l'on néglige les éventuelles dérives temporelles, le procédé comporte un paramétrage d'un modèle de calibration. Le modèle de calibration est configuré pour recevoir des données d'entrée, correspondant à des mesures acquises *X(t)* par le capteur de mesure à un instant de mesure *t*. Il est également configuré pour estimer des mesures de référence *Z*(*t*) à partir des données d'entrée. Par mesures de référence, on entend des mesures calibrées, de telle sorte que chaque mesure calibrée correspond à une grandeur physique mesurée, indépendamment du type ou du fabricant du capteur l'ayant enregistrée, dans un espace de référence. Ainsi, l'application du modèle de calibration peut être considérée comme correspondant à un changement de référentiel, entre un référentiel initial, correspondant au capteur de mesure, et un référentiel final. Dans le référentiel final, deux mesures, correspondant à une même grandeur physique ou chimique, et mesurées respectivement par deux capteurs de mesure différents, ont une même valeur, ou une valeur considérée comme identique.

Lorsque la calibration est effectuée en prenant en compte un capteur de référence, le modèle de calibration permet d'estimer, à partir d'une mesure effectuée par un capteur de mesure 2, porté par un utilisateur, une mesure qui aurait été délivrée par le capteur de référence, mesurant la même grandeur physique ou chimique.

Lorsque la calibration est effectuée sans capteur de référence, mais en prenant en compte un fantôme dont maîtrise l'évolution d'une grandeur physique, une valeur de référence est considérée comme correspondant à ladite grandeur physique.

Une mesure *X(t)* délivrée par un capteur de mesure 2 prend la forme d'un scalaire ou d'un vecteur, le vecteur ayant différentes coordonnées. Une mesure de référence *Z*(*t*), exprimée dans le référentiel final, prend également une forme de scalaire ou d'un vecteur. Cependant, pour une même grandeur physique mesurée par différents capteurs de mesure, différents les uns des autres, le modèle de calibration permet d'obtenir des valeurs de référence identiques, ou pouvant être considérées comme telles.

Le modèle de calibration est établi au cours de la phase de calibration, ou phase d'apprentissage, à l'aide des séquences de calibration et des séquences de référence qui leurs sont respectivement associées. Il existe plusieurs façons de déterminer le modèle de calibration. Les inventeurs ont considéré qu'il était préférable que le modèle de calibration soit établi à l'aide d'un algorithme d'intelligence artificielle supervisé, et par exemple en utilisant un réseau de neurones.

Par algorithme d'intelligence artificielle supervisé, on entend un algorithme paramétré durant une phase d'apprentissage, au cours de laquelle on dispose de données d'entrée et de données de sorties maîtrisées. Dans le cas présent, l'apprentissage est effectué en utilisant les séquences de calibration en tant que données d'entrée de l'algorithme et les séquences de référence en tant que données de sortie de l'algorithme.

Dans l'application visée, les données d'entrée et de sortie prennent la forme de séquences de données temporelles. Le recours à des réseaux de neurones récurrents, de préférence bidirectionnels, est donc particulièrement approprié.

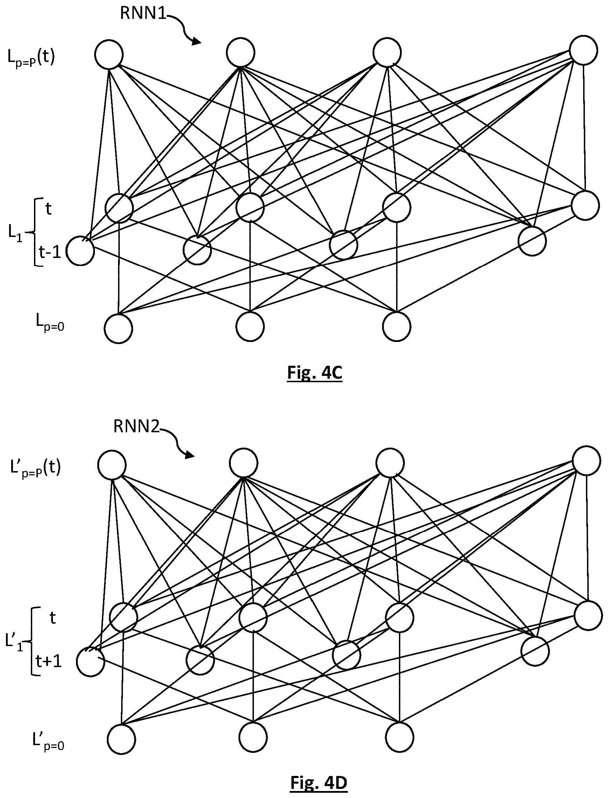

On a schématisé, sur la figure 4C, un premier réseau de neurones récurrent RNN1, connu de l'homme du métier, et usuellement désigné par l'acronyme RNN (Recurrent Neural Network). Les réseaux de neurones récurrents sont connus de l'homme du métier, en particulier pour des applications liées à la reconnaissance vocale ou la traduction automatique. Un exemple d'application est donné dans la publication Cho K."Learning Phase Representations using RNN Encoder-Decoder for Statistical Machine Translation" *ArXiv* abs/1406.1078 (2014). Dans l'exemple représenté sur la figure 4C, le réseau de neurones récurrent comporte une couche d'entrée comportant les mesures *Xₖ*(*t*) réalisées par le capteur de mesure à un instant de calibration t Le réseau comporte également une couche cachée L₁ et une couche de sortie L_{P}. P est un entier strictement supérieur à 1 désignant le nombre de couches s'ajoutant à la couche d'entrée. La couche d'entrée *Xₖ*(*t*) correspond à chaque mesure acquise à un instant de mesure *t.* La dimension de *Xₖ*(*t*) correspond à la dimension de chaque mesure. Par exemple, lorsque chaque mesure est une accélération selon trois axes, la dimension de *Xₖ*(*t*) est égale à 3. Lorsque chaque mesure est un scalaire, la dimension de *Xₖ*(*t*) est égale à 1.

Pour une séquence de vecteurs d'entrée *Xₖ*(*t*) , avec *t* ∈ [*t*₁ ... . *t_{N}*], on obtient une séquence de vecteurs de sortie *Xₖ*(*t*) , avec *t* ∈ [*t*₁ *.... t_{N}*]. Entre la couche d'entrée et la couche de sortie s'étend au moins une couche cachée *Lₚ,* avec 0<p<P. La couche *L_{p=P}* correspond à la couche de sortie.

Dans le cas de réseaux de neurones récurrents, la valeur des neurones de chaque couche cachée *Lₚ*(*t*), à chaque instant de calibration *t,* peut être obtenue :
- soit en considérant un ordre chronologique croissant, en considérant ladite couche à un instant *t -* 1 antérieur à l'instant *t,* ce qui correspond au réseau de neurones RNN1 représenté sur la figure 4C ;
- soit en considérant un ordre chronologique décroissant, en considérant ladite couche à un instant *t +* 1 postérieur à l'instant *t,* ce qui correspond au réseau de neurones RNN2 représenté sur la figure 4D.

Lorsqu'on considère l'ordre chronologique croissant, ce qui correspond au premier réseau de neurones RNN1 schématisé sur la figure 4C, le contenu *Lₚ*(*t*) de chaque couche de rang p, avec 1≤p≤P est déterminé en prenant en compte :
- une fonction de transfert ${σ}_{p} : {ℝ}^{{n}_{p}} \to {ℝ}^{{n}_{p}}$*, nₚ* étant le nombre de neurones de la couche *Lₚ*(*t*). La fonction de transfert *σₚ* comporte *nₚ* fonctions d'activation élémentaires $ℝ \to ℝ$. Chaque fonction élémentaire est généralement non linéaire et bornée. Il s'agit usuellement d'une fonction de type tangente hyperbolique ou une fonction sigmoïde.
- *Wₚ* est une première matrice d'interconnexion définie pour chaque couche *Lₚ*(*t*), permettant un passage de la couche *L*_{*p-*1}(*t*) à la couche *Lₚ*(*t*), de dimension [*n*_{*p*-1}, *nₚ*], et dont chaque terme est correspond à un poids.
- *Yₚ* est une deuxième matrice d'interconnexion définie pour chaque couche *Lₚ,* permettant un passage de la couche *Lₚ* (*t -* 1) à la couche *Lₚ*(*t*), de dimension [*nₚ, nₚ*], et dont chaque terme est correspond à un poids.
- *bₚ* est un vecteur de biais de la couche *Lₚ,* de dimension [1, *nₚ*].

La valeur de chaque couche *Lₚ*(*t*) est mise à jour à chaque instant de mesure, et peut être représentée par un vecteur de dimension [1, *nₚ*]. Elle est obtenue à partir d'une couche précédente *L*_{*p*-1}(*t*) à l'instant de mesure, et à partir de la couche *Lₚ*(*t -* 1) de même rang p, à un instant *t* - 1 précédent l'instant de mesure, selon l'expression : ${L}_{p} \left(t\right) = {σ}_{p} \left[{W}_{p}\times {L}_{p - 1}\left(t\right)+{b}_{p}+{Y}_{p}\times {L}_{p}\left(t-1\right)\right]$

Lors de la calibration, la couche d'entrée Lₚ₌₀ est Xₖ(t).

Lorsqu'on prend en compte l'ordre chronologique décroissant, on utilise un deuxième réseau de neurones récurrent RNN2, ayant une structure telle que celle représentée sur la figure 4D. Le contenu de chaque couche *L'ₚ*(*t*) est calculé, à un instant t, selon l'expression suivante, analogue à l'expression (3): $L {′}_{p} \left(t\right) = σ {′}_{p} \left[W{′}_{p}\times L{′}_{p - 1}\left(t\right)+b{′}_{p}+Y{′}_{p}\times L{′}_{p}\left(t-1\right)\right]$ où *W'ₚ, Y'ₚ, b'ₚ* et *σ'ₚ* sont respectivement une première matrice de connexion, une deuxième matrice de connexion, un vecteur de biais et une fonction d'activation respectivement analogues à la première matrice de connexion, la deuxième matrice de connexion, le vecteur de biais et aux fonctions d'activation décrits en lien avec l'expression (3). Lors de la calibration, la couche d'entrée L'ₚ₌₀ est Xₖ(t).

Les expressions (3) et (4) supposent une initialisation, en considérant la valeur de chaque couche égale à une valeur prédéterminée, ou à une valeur aléatoire, à t = 0.

Le premier réseau de neurones RNN1 et le deuxième réseau de neurones RNN2 peuvent comprendre entre 1 et 3 couches cachées, entre la couche d'entrée et de sortie. Le nombre de neurones de chaque couche cachée peut être compris entre 10 et 100, voire davantage.

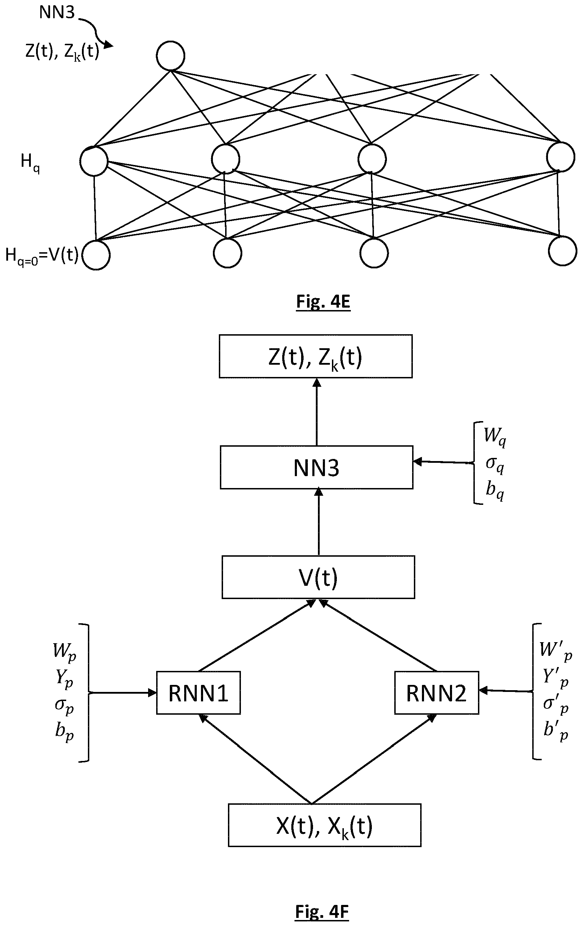

On forme ensuite un vecteur *V*(*t*) en concaténant les couches de sortie *L_{p=P}*(*t*) et *L'_{p=P}*(*t*). Le vecteur *V*(*t*) est utilisé en tant que donnée d'entrée H_{q=0} d'un troisième réseau de neurones NN3, schématisé sur la figure 4E. Le troisième réseau de neurones NN3 comporte au moins une couche cachée H_{q} ainsi qu'une couche de sortie H_{q=Q}. L'indice q est un entier désignant le rang de chaque couche. Q est un entier strictement supérieur à 1 désignant le nombre de couches en dessus de la couche d'entrée. Le troisième réseau de neurones NN3 peut comprendre entre 1 et 3 couches cachées, entre la couche d'entrée et de sortie. Le nombre de neurones de chaque couche cachée peut être compris entre 10 et 100, voire davantage.

Le troisième réseau de neurones est un réseau de neurones standard, i-e non récurrent, connu de l'homme du métier. Le contenu de chaque couche de rang q, avec 1<q≤Q est déterminé en prenant en compte :
- une fonction de transfert ${σ}_{q} : {ℝ}^{{n}_{q}} \to {ℝ}^{{n}_{q}}$, *n_{q}* étant le nombre de neurones de la couche *H_{q}.* La fonction de transfert *σ_{q}* comporte *n_{q}* fonctions de transfert élémentaires $ℝ \to ℝ$. Chaque fonction élémentaire est généralement non linéaire, bornée et dérivable. Il s'agit usuellement d'une fonction de type tangente hyperbolique ou une fonction sigmoïde ;
- une matrice d'interconnexion *W_{q}* définie pour chaque couche *H_{q},* permettant un passage de la couche *H*_{*q*-1} à la couche *H_{q},* de dimension [*n*_{*q*-1}, *n_{q}*], et dont chaque terme est correspond à un poids.
- un vecteur de biais *b_{q}* de la couche *H_{q},* de dimension [1, *n_{q}*].

Le contenu de chaque couche *H_{q}* est calculé selon l'expression suivante : ${H}_{q} = {σ}_{q} \left[{W}_{q}\times {H}_{q - 1}+{b}_{q}\right]$

Lors de la calibration, La couche de sortie *H_{q=Q}* du troisième réseau de neurones NN3 est une mesure de référence, également notée Zₖ(t), correspondant à la mesure de calibration Xₖ(t) formant la couche d'entrée du premier réseau de neurones RNN1 et du deuxième réseau de neurones RNN2.

La figure 4F représente l'articulation entre les différents réseaux de neurones, formant le modèle de calibration.

La phase d'apprentissage permet de définir les paramètres du modèle d'activation. Il s'agit, pour chaque couche d'ordre p≥1, des paramètres régissant chaque réseau de neurones, c'est-à-dire :
- les matrices *Wₚ, Yₚ, W'ₚ , Y'ₚ, W_{q}* ;
- les fonctions de transfert *σₚ, σ'ₚ, σ_{q}* ;
- les vecteurs *bₚ, b'ₚ, b_{q}.*

Lors de l'apprentissage, la couche d'entrée est formée à partir de mesures de calibration *Xₖ*(*t*) formant chaque séquence de calibration, et la couche de sortie est formée à partir de mesures de référence *Zₖ*(*t*) formant chaque séquence de référence correspondant respectivement à la séquence de calibration utilisée pour définir la couche d'entrée. De préférence, l'apprentissage requiert un grand nombre de séquences de calibrations.

L'apprentissage consiste à déterminer les paramètres du modèle de calibration permettant d'obtenir la meilleure estimation possible de chaque mesure de référence, à partir des mesures de calibration. Pour cela, on peut se baser sur une fonction de coût *Cost,* cette dernière correspondant à une erreur quadratique moyenne (RMSE) ou une erreur absolue moyenne (MAE) entre les mesures de référence respectivement mesurées et estimées par le modèle de calibration. Lorsqu'on se base sur l'erreur absolue moyenne, les paramètres du modèle sont ceux minimisant une fonction de coût *Cost* telle que : $Cost = {\sum }_{t , k} \left|{Z}_{k}\left(t\right)-{\hat{Z}}_{k}\left(t\right)\right|$ où *Zₖ*(*t*) et *Ẑₖ*(*t*) sont respectivement une mesure de référence obtenue et estimée à un instant de calibration *t* d'une séquence de calibration *k.*

Selon un autre mode de réalisation, le modèle de calibration est un modèle linéaire. Il peut s'agir d'une matrice de passage *A*, de dimension (nₓ, nₓ), où nₓ correspond à la dimension de chaque mesure. La matrice de passage est déterminée par apprentissage, en se basant par exemple sur une minimisation d'une fonction de coût comme décrit en lien avec l'expression (6). Les mesures de référence peuvent être telles que *2*(*t*) *= AX*(*t*). La matrice *A* peut être définie de telle sorte que : $A = arg {min}_{A} {\sum }_{t , k} \left|{Z}_{k}\left(t\right)-{\hat{Z}}_{k}\left(t\right)\right|$

Cependant, les inventeurs estiment qu'il est préférable de recourir à un modèle de calibration non linéaire, de préférence établi à partir d'un algorithme d'intelligence artificielle supervisée.

### Etape 230 : utilisation du modèle de calibration.

Après que le modèle de calibration, schématisé sur la figure 4F a été déterminé (cf. étape 220), il est mis en œuvre pour estimer des mesures de référence *Z*(*t*)*,* à un instant de mesure *t,* à partir de mesures *X(t)* acquises par le capteur de mesure. Les grandeurs *Z*(*t*) et *X*(*t*) ont la même dimension. Il s'agit de grandeurs vectorielles ou scalaires. Lors de l'utilisation du modèle de calibration, la couche d'entrée du modèle est X(t), tandis que la couche de sortie est Z(t).

### Exemples.

On décrit ci-après des exemples expérimentaux de traitement de mesures, acquises par des capteurs de mouvement, par un modèle de calibration tel que décrit en lien avec la figure 4F.

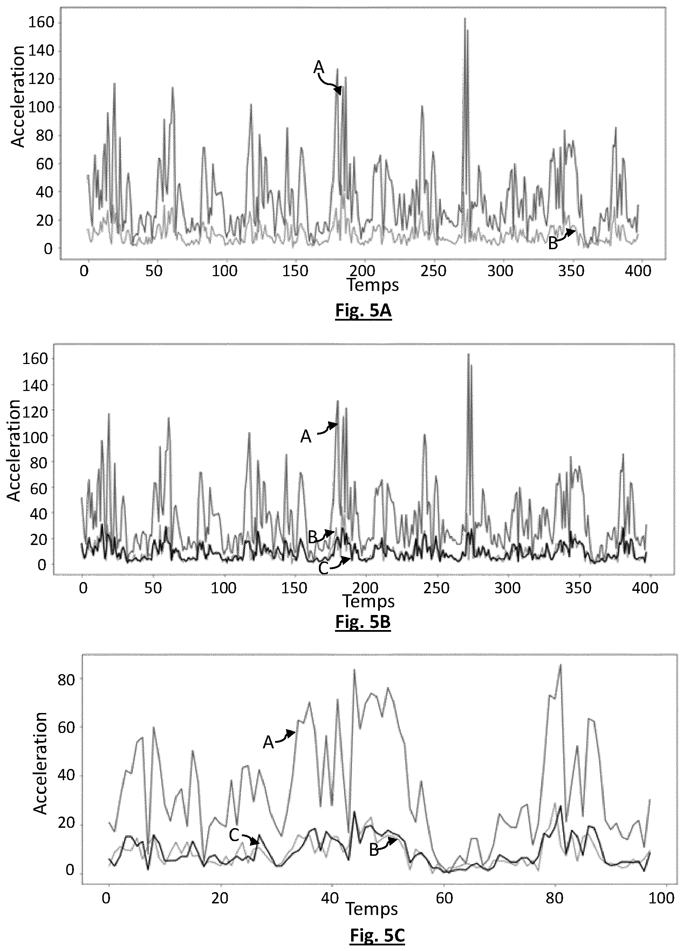

Dans une première série d'essais, on a utilisé deux capteurs de mouvement identiques (capteur A et capteur B), comportant des accéléromètres. Les capteurs utilisés sont des centrales inertielles pour téléphones portables ST LSM6DSM (fabricant ST Microelectronics). Les logiciels de paramétrage de chaque capteur ont été configurés de façon que les filtres passe-bas soient paramétrés différemment. Les capteurs ont été disposés sur le même poignet d'un utilisateur. La figure 5A montre des mesures d'accélération, selon un axe, acquises à différents instants de mesure. Le capteur A été considéré comme étant un capteur de référence. Le capteur B a été considéré comme étant le capteur devant faire l'objet d'une calibration. Les mesures représentées sur la figure 5A ont été utilisées en tant que mesures de calibration (capteur B) et de référence (capteur A) pour établir le modèle de calibration.

Pour cet exemple, et à titre non limitatif, le modèle de calibration était tel que :
- le premier réseau de neurones RNN1 comporte une couche cachée, comprenant 50 neurones ;
- le deuxième réseau de neurones RNN2 comporte une couche cachée, comprenant 50 neurones ;
- le troisième réseau de neurones NN3 comporte une couche cachée, comprenant 3 neurones ;

La figure 5B représente :
- des mesures de référence, acquises par le capteur A (courbe A) ;
- des mesures acquises par le capteur B à calibrer (courbe B) ;
- des mesures acquises par le capteur B et traitées par le modèle de calibration (courbe C).

On observe la cohérence entre les mesures de référence (courbe A) et leurs estimations par le modèle de calibration (courbe C).

La figure 5C est un détail de la figure 5B, dans la plage temporelle 300s - 400s.

Dans une deuxième série d'essais, on a utilisé deux capteurs de mesure différents : un capteur geneactiv (GE)- fabricant Active Insights (GE) et une montre applewatch - fabricant Apple (AW). Au cours de différents instants, on a mesuré un vecteur d'accélération tri axes. Dans la suite, le capteur de référence est le capteur GE. Le capteur AW est le capteur faisant l'objet de la calibration.

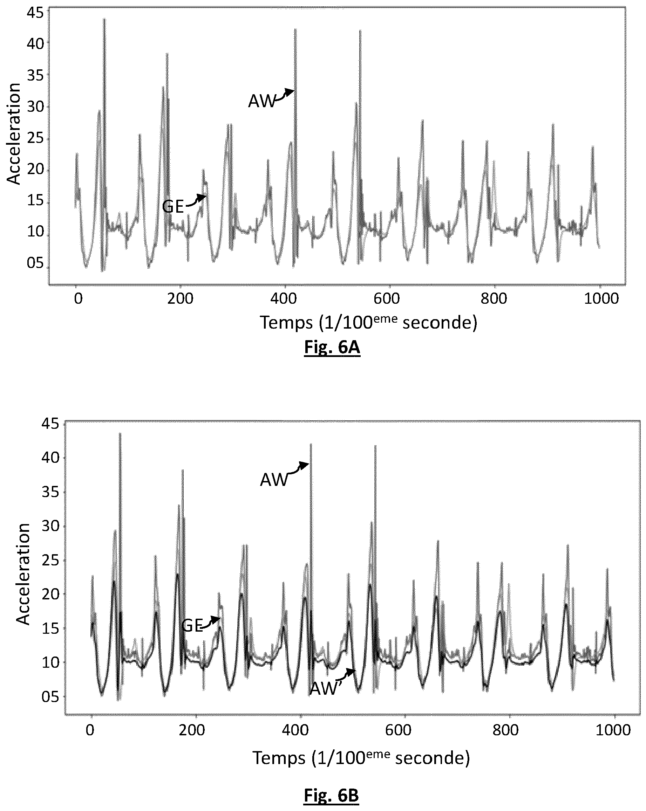

La figure 6A montre des mesures d'accélération, selon un axe, acquises à différents instants de mesure. Les capteurs étaient disposés sur le poignet d'un utilisateur, ce dernier effectuant une marche. Les mesures représentées sur la figure 6A ont été utilisées pour établir le modèle de calibration.

Le modèle de calibration était tel que :
- le premier réseau de neurones RNN1 comporte deux couches cachées, chaque couche cachée comprenant 50 neurones ;
- le deuxième réseau de neurones RNN2 comporte deux couches cachées, chaque couche cachée comprenant 50 neurones ;
- le troisième réseau de neurones NN3 comporte trois couches cachées, chaque couche cachée comprenant 20 neurones, 20 neurones et 3 neurones respectivement ;

La figure 6B représente :
- des mesures de référence, acquises par le capteur GE (courbe GE) ;
- des mesures acquises par le capteur AW à calibrer (courbe AW) ;
- des mesures acquises par le capteur AW et traitées par le modèle de calibration (courbe AW').

L'erreur moyenne quadratique (RMSE) a été calculée :
- ente les mesures de référence (GE) et les mesures acquises par le capteur AW : RMSE = 0.316;
- entre les mesures de référence (courbe GE) et leurs estimations par le modèle de calibration (courbe AW') : RMSE = 0.139.

La comparaison entre les RMSE montre que le traitement des données mesurées par le capteur AW permet de se rapprocher significativement des mesures de référence, ce qui atteste de la pertinence de l'invention.

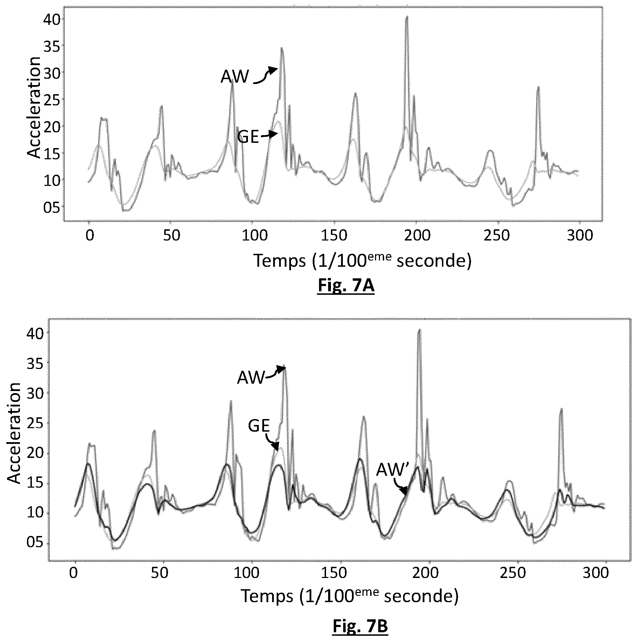

Les capteurs GE et AW ont été utilisés pour acquérir des mesures lorsque l'utilisateur courait. La figure 7A montre des mesures d'accélération, selon un axe, acquises à différents instants de mesure. Les mesures représentées sur la figure 7A ont été utilisées pour établir le modèle de calibration.

La figure 7B représente :
- des mesures de référence, acquises par le capteur GE (courbe GE) ;
- des mesures acquises par le capteur AW à calibrer (courbe AW) ;
- des mesures acquises par le capteur AW et traitées par le modèle de calibration (courbe AW').

L'erreur moyenne quadratique (RMSE) a été calculée :
- ente les mesures de référence et les mesures acquises par le capteur AW : RMSE = 0.267;
- entre les mesures de référence (courbe GE) et leurs estimations par le modèle de calibration (courbe AW') : RMSE = 0.126.

Dans une autre série d'essais, on a testé une mise en œuvre des étapes 100 à 150 décrites en lien avec la figure 3A. On a utilisé des centrales inertielles (IMU Inertial Module Unit) référence iNEMO fournisseur ST Micro, faisant office de capteurs. Chaque centrale inertielle a été montée sur un bracelet, relié au poignet d'un utilisateur de test. Chaque centrale inertielle comportait un accéléromètre, ainsi que des registres permettant le réglage des valeurs des paramètres de l'accéléromètre. Plus précisément, on a utilisé :
- un dispositif de référence, comportant la centrale inertielle dont les registres ont été paramétrés pour correspondre à une configuration de référence.
- un dispositif de test, comportant une autre centrale inertielle, identique à la précédente, dont les registres ont été paramétrés de façon différente par rapport à la configuration de référence.

Le dispositif de test et le dispositif de référence ont été portés au même poignet d'un même utilisateur.

Parmi les registres reliés à l'accéléromètre, certains registres permettent une configuration d'un filtre appliqué aux mesures résultant de l'accéléromètre. Les paramètres sont :
- HP_SLOPE_XL_EN : lorsque la valeur est fixée à 0, le filtre est un filtre passe bas. Lorsque la valeur est fixée à 1, le filtre est un filtre passe-haut.
- LPF2_XL_EN , LPF1_BW_ESL et HPCF_XL : ces registres permettent de définir les paramètres de filtrage, et plus précisément la fréquence de coupure. La fréquence de coupure est déterminée en fonction de la fréquence d'échantillonnage ODR (Output Data Rate).

La figure 8A représente des mesures effectuées par l'utilisateur, alors que ce dernier marchait. Le dispositif de référence (ref) et le dispositif de test (test) étaient paramétrés selon la configuration de référence. L'axe des abscisses correspond au temps (unité secondes) et l'axe des ordonnées correspond aux mesures d'accélération (unité mg où g correspond à l'intensité de l'accélération de pesanteur). Le tableau 1 montre la valeur de chaque paramètre dans la configuration de référence (cf. deuxième colonne). Le symbole « -» signifie valeur indifférente.

**Tableau 1**

| Registres | Valeurs de référence | Valeurs modifiées |
|---|---|---|
| HP_SLOPE_XL_EN | 1 | 1 |
| LPF2_XL_EN | 0 | 1 |
| LPF1_BW_ESL | - | - |
| HPCF_XL | - | 01 |
| Fréquence de coupure | ODR/2 | ODR/100 |

Sur la figure 8A, les courbes « ref » et « test », correspondant respectivement au dispositif de référence et au dispositif de test, se confondent.

La figure 8B représente des mesures effectuées par l'utilisateur, ce dernier poursuivant son activité de marche en portant le dispositif de référence ainsi que le dispositif de test. La configuration de ce dernier, c'est-à-dire le paramétrage des registres, a été modifiée, entraînant une modification de la fréquence de coupure. Cf. colonne de droite du tableau 1. Sur la figure 8B, l'axe des abscisses correspond au temps (unité secondes) et l'axe des ordonnées correspond aux mesures d'accélération (unité mg où g correspond à la force de pesanteur).

Les erreurs quadratiques des mesures (RMSE) reportées sur les figures 8A et 8B ont été calculées. Les RMSE sont respectivement égales à 9 mg et à 25.1 mg. La comparaison entre les figures 8A et 8B met en évidence l'effet, sur les mesures, d'un paramétrage incorrect du capteur de mesure. C'est ce que l'invention permet de résoudre.

La figure 8C est une simulation de mise en œuvre de l'invention. L'axe des ordonnées correspond à l'accélération (unité mg). L'axe des abscisses correspond au temps (unité millisecondes). La courbe a) est représentative d'un fonctionnement nominal du capteur, c'est-à-dire paramétré selon des valeurs spécifiées. Entre t = 580 ms et t = 720 ms, on a simulé un déréglage intempestif de la valeur d'un paramètre : alors que le capteur était correctement paramétré, la valeur d'un paramètre du capteur est modifiée à 580 ms. Une vérification de la valeur du paramètre est intervenue à t = 720 ms. La vérification étant non conforme, une correction de la valeur du paramètre a été effectuée, de façon que le paramètre reprenne la valeur initiale. La période comprise entre 580 ms et 720 ms montre l'effet d'un déréglage intempestif d'une valeur d'un paramètre : cf. courbe b). La figure 8C montre l'effet de l'invention, avant et après 720 ms. L'invention permet de réajuster la valeur incorrecte du paramètre à la valeur initiale, ce qui conduit à des mesures représentatives du fonctionnement nominal du capteur.

L'invention permet d'obtenir des mesures, représentatives d'une évolution temporelle d'une grandeur physique, indépendamment des capteurs permettant l'acquisition des mesures. Les mesures, après la calibration telle que précédemment décrites, sont comparables entre elles, ou « standardisées ». Elles peuvent être exploitées par une même application d'interprétation.

Dans les exemples qui précèdent, l'application d'interface 7 est mise en œuvre par un microprocesseur, en l'occurrence l'unité centrale 5, intégré dans le même dispositif 1 que le capteur de mesure. Selon un autre mode de réalisation, l'application d'interface 7 est mise en œuvre par un microprocesseur distant. Selon ce mode de réalisation, représenté sur la figure 9A, les données mesurées par le capteur 2 sont transmises à un microprocesseur distant 7', mettant en œuvre l'application d'interface. L'application d'interface 7 peut être mise en œuvre dans un serveur accessible par internet, de type cloud. Les mesures de référence issues de l'application d'interface sont ensuite transmises à un autre microprocesseur distant 6', mettant en œuvre l'application d'interprétation 6. A l'instar de l'application d'interface 7, l'application d'interprétation peut être mise en œuvre dans un serveur accessible par internet, de type cloud.

Dans ce cas, les mesures transmises par le capteur de mesure comportent un identifiant, permettant à l'application d'interface 7 d'identifier le capteur de mesure et d'appliquer un modèle de calibration correspondant à ce dernier, préalablement établi au cours d'une phase de calibration et mémorisé dans une mémoire reliée au microprocesseur distant 7'.

Selon un autre mode de réalisation, l'application d'interface est mise en œuvre dans le même dispositif que celui comportant le capteur de mesure. L'application d'interprétation est mise en œuvre par un microprocesseur distant 6'. Une telle variante est représentée sur la figure 9B.

## Revendications

1. Procédé de traitement de mesures, acquises par un capteur de mesure (2), à différents instants de mesure, le capteur de mesure étant:
- intégré dans un dispositif connecté , porté par un utilisateur, le dispositif connecté étant configuré pour être relié à un réseau de communication sans fil ;
- configuré pour acquérir, à chaque instant de mesure, une mesure (*X*(*t*)) représentative d'un mouvement de l'utilisateur ou d'une caractéristique physiologique de l'utilisateur ;
- relié à un circuit électronique de commande, configuré pour commander une acquisition de mesures par le capteur, et/ou un prétraitement de mesures acquises par le capteur ;
- paramétré par des paramètres du capteur, chaque paramètre du capteur étant mémorisé dans un registre de commande du circuit électronique de commande ;
le procédé comportant :
- une acquisition de mesures d'une grandeur chimique ou physique par le capteur de mesure à différents instants de mesure, de façon à former une séquence de mesures ;
- une transmission de données, établies à partir de la séquence de mesures, à une application d'interprétation (6), l'application d'interprétation étant programmée pour estimer, à partir des données transmises, un état de l'utilisateur, l'état de l'utilisateur étant choisi parmi plusieurs états prédéterminés;
le procédé comportant, suite à l'acquisition de la séquences de mesure :
- traitement de chaque mesure (*X*(*t*)) de la séquence de mesures par un modèle de calibration, de façon à estimer, à partir de chaque mesure, une mesure de référence *(Z(t));*
- transmission de chaque mesure de référence estimée à l'application d'interprétation (6), les mesures de référence formant alors les données transmises à l'application d'interprétation ;
le procédé étant **caractérisé en ce que** le modèle de calibration est établi au cours d'une phase de calibration, comportant différents instants de calibration, la phase de calibration comprenant:
- i) acquisition de mesures de calibration (*Xₖ*(*t*)) par le capteur de mesure, aux différents instants de calibration, et obtention de mesures de référence (*Zₖ*(*t*)), à chaque instant de calibration, de telle sorte qu'à chaque mesure de calibration correspond une mesure de référence, au moins une mesure de référence étant représentative d'un état de l'utilisateur parmi les états prédéterminés, les mesures de calibration et les mesures de référence mesurant la même grandeur chimique ou physique;
- ii) à partir des mesures de référence (*Zₖ*(*t*)) et des mesures de calibration (*Xₖ*(*t*)), définition d'un modèle de calibration, le modèle de calibration étant configuré pour estimer des mesures de référence à partir de mesures acquises par le capteur de mesure.

2. Procédé selon la revendication 1, dans lequel lors de l'étape i), le capteur de mesure (2) est disposé sur un fantôme, représentatif d'au moins un état de l'utilisateur, les mesures de référence étant obtenues à partir du fantôme, configuré pour faire varier la grandeur physique dans une plage de mesure cohérente avec des valeurs susceptibles d'être mesurées dans des conditions réelles.

3. Procédé selon la revendication 2, dans lequel le fantôme comporte un capteur de référence, le capteur de référence délivrant une mesure de référence à chaque instant de calibration.

4. Procédé selon la revendication 1, dans lequel lors de l'étape i), le capteur de mesure est disposé sur au moins un individu test, l'individu test portant également un capteur de référence, le capteur de référence délivrant une mesure de référence à chaque instant de calibration.

5. Procédé selon l'une quelconque des revendications précédentes, dans lequel le modèle de calibration met en œuvre un algorithme d'intelligence artificielle supervisé, l'algorithme d'intelligence artificielle supervisé étant paramétré au cours de la phase de calibration.

6. Procédé selon la revendication 5, dans lequel l'algorithme d'intelligence artificielle supervisé comporte un réseau de neurones.

7. Procédé selon la revendication 6, dans lequel le réseau de neurones est un réseau de neurones récurrent, et de préférence bidirectionnel, le réseau de neurones comportant une couche d'entrée et une couche de sortie, de telle sorte qu'au cours du traitement de chaque mesure par le modèle de calibration :
- chaque mesure acquise (*X*(*t*)) par le capteur de mesure forme la couche d'entrée du réseau de neurones ;
- la couche de sortie correspond à l'estimation (*Z*(*t*)) d'au moins une mesure de référence.

8. Procédé selon l'une quelconque des revendications précédentes comportant, entre les étapes i) et ii), une synchronisation temporelle des mesures de calibration par rapport aux mesures de référence.

9. Procédé selon l'une quelconque des revendications précédentes, dans lequel le capteur comporte au moins :
- un capteur de mouvement, de type accéléromètre et/ou gyromètre et/ou magnétomètre;
- et/ou un capteur de pression ;
- et/ou un capteur optique ;
- et/ou un capteur électrique ;
- et/ou un capteur chimique ;
- et/ou un capteur de température ;
- et/ou un capteur physiologique, configuré pour déterminer une caractéristique physiologique de l'utilisateur ;.

10. Procédé selon l'une quelconque des revendications précédentes, dans lequel l'état de l'utilisateur est choisi parmi :
- un état décrivant une activité physique de l'utilisateur ;
- un état de stress ;
- un état de sommeil ou de somnolence ;
- un état pathologique ;
- un état symptomatique ;
- un état correspondant à une survenue d'une situation à risque pour l'utilisateur.

11. Procédé selon l'une quelconque des revendications précédentes, dans lequel l'utilisateur est un être humain vivant ou un animal vivant.

12. Procédé selon l'une quelconque des revendications précédentes, dans lequel l'application d'interprétation est mise en œuvre par un microprocesseur intégré dans le dispositif connecté, ou par un microprocesseur distant du dispositif connecté, et relié à ce dernier par une liaison filaire ou sans fil.

13. Dispositif connecté (1), destiné à être porté par un utilisateur, comportant un capteur de mesure (2), le capteur de mesure étant configuré pour acquérir, à différents instants de mesure, une mesure (X(t)) d'une grandeur chimique ou physique représentative d'un mouvement de l'utilisateur ou d'une caractéristique physiologique de l'utilisateur, le capteur de mesure étant paramétré par des paramètres du capteur, chaque paramètre du capteur étant mémorisé dans un registre de commande d'un circuit de commande du capteur, le dispositif étant configuré pour activer une application d'interprétation (6), l'application d'interprétation étant programmée pour estimer, à partir des mesures acquises par le capteur de mesure, un état de l'utilisateur, l'état de l'utilisateur étant choisi parmi plusieurs états prédéterminés, le dispositif étant **caractérisé en ce qu'**il comporte une unité centrale (5), programmée pour:
- traiter de chaque mesure par un modèle de calibration de façon à obtenir, à partir de chaque mesure, une mesure de référence ;
- transmettre chaque mesure de référence à l'application d'interprétation ;
- l'unité centrale étant configurée de façon que le modèle de calibration est obtenu au cours d'une phase de calibration, comportant différents instants de calibration, la phase de calibration comprenant les étapes i) et ii) de l'une quelconque des revendications précédentes.

## Patentansprüche

1. Verfahren zur Verarbeitung von Messungen, die von einem Messsensor (2) zu verschiedenen Messzeitpunkten erfasst werden, wobei der Messsensor:
- in eine vernetzte Vorrichtung (1) integriert ist, die von einem Benutzer getragen wird, wobei die vernetzte Vorrichtung dazu ausgestaltet ist, mit einem Drahtloskommunikationsnetz verbunden zu werden;
- dazu ausgestaltet ist, zu jedem Messzeitpunkt eine Messung (*X*(*t*)) zu erfassen, die für eine Bewegung des Benutzers oder für ein physiologisches Merkmal des Benutzers repräsentativ ist;
- mit einer elektronischen Steuerschaltung verbunden ist, die dazu ausgestaltet ist, eine Erfassung von Messungen durch den Sensor und/oder eine Vorverarbeitung von von dem Sensor erfassten Messungen zu steuern;
- durch Parameter des Sensors parametriert wird, wobei jeder Parameter des Sensors in einem Steuerregister der elektronischen Steuerschaltung gespeichert wird; wobei das Verfahren umfasst:
- ein Erfassen von Messungen durch den Messsensor zu verschiedenen Messzeitpunkten, so dass eine Messsequenz gebildet wird;
- eine Übertragung von Daten, die ausgehend von der Messsequenz erstellt werden, an eine Auswerteapplikation (6), wobei die Auswerteapplikation dazu programmiert ist, ausgehend von den übertragenen Daten einen Zustand des Benutzers zu schätzen, wobei der Zustand des Benutzers unter mehreren vorbestimmten Zuständen gewählt wird;
wobei das Verfahren umfasst, nach dem Erfassen einer Messsequenz:
- Verarbeitung jeder Messung (*X(t)*) der Messsequenz durch ein Kalibrierungsmodell, so dass ausgehend von jeder Messung eine Referenzmessung (*Z(t)*) geschätzt wird;
- Übertragung jeder geschätzten Referenzmessung an die Auswerteapplikation (6), wobei die Referenzmessungen dann die an die Auswerteapplikation übertragenen Daten bilden;
wobei das Verfahren **dadurch gekennzeichnet ist, dass** das Kalibrierungsmodell während einer Kalibrierungsphase erstellt wird, die verschiedene Kalibrierungszeitpunkte umfasst, wobei die Kalibrierungsphase umfasst:
- i) Erfassen von Kalibrierungsmessungen (*Xₖ(t)*) durch den Messsensor zu den verschiedenen Kalibrierungszeitpunkten und Erhalten von Referenzmessungen (*Zₖ(t)*) für jeden Kalibrierungszeitpunkt, so dass jeder Kalibrierungsmessung eine Referenzmessung entspricht, wobei mindestens eine Referenzmessung für einen Zustand des Benutzers unter den vorbestimmten Zuständen repräsentativ ist, wobei die Kalibrierungsmessungen und die Referenzmessungen dieselbe physikalische oder chemische Grösse messen ;
- ii) ausgehend von den Referenzmessungen (*Zₖ(t)*) und den Kalibrierungsmessungen (*Xₖ(t)*)*,* Definition eines Kalibrierungsmodells, wobei das Kalibrierungsmodell dazu ausgestaltet ist, Referenzmessungen ausgehend von dem Messsensor erfassten Messungen zu schätzen.

2. Verfahren nach Anspruch 1, bei dem beim Schritt i) der Messsensor (2) auf einem Phantom angeordnet wird, das für mindestens einen Zustand des Benutzers repräsentativ ist, wobei die Referenzmessungen ausgehend von dem Phantom erhalten werden, wobei das Phantom so konfiguriert ist, dass die physikalische Größe innerhalb eines Messbereichs variiert wird, der den unter realen Bedingungen zu erwartenden Messwerten entspricht.

3. Verfahren nach Anspruch 2, bei dem das Phantom einen Referenzsensor umfasst, wobei der Referenzsensor zu jedem Kalibrierungszeitpunkt eine Referenzmessung ausgibt.

4. Verfahren nach Anspruch 1, bei dem beim Schritt i) der Kalibrierungssensor auf mindestens einer Versuchsperson angeordnet wird, wobei die Versuchsperson auch einen Referenzsensor trägt, wobei der Referenzsensor zu jedem Kalibrierungszeitpunkt eine Referenzmessung ausgibt.

5. Verfahren nach einem der vorhergehenden Ansprüche, bei dem das Kalibrierungsmodell einen überwachten Algorithmus der künstlichen Intelligenz ausführt, wobei der überwachte Algorithmus der künstlichen Intelligenz während der Kalibrierungsphase parametriert wird.

6. Verfahren nach Anspruch 5, bei dem der überwachte Algorithmus der künstlichen Intelligenz ein neuronales Netz umfasst.

7. Verfahren nach Anspruch 6, bei dem das neuronale Netz ein rekurrentes und bevorzugt bidirektionales neuronales Netz umfasst, wobei das neuronale Netz eine Eingabeschicht und eine Ausgabeschicht umfasst, so dass während der Verarbeitung jeder Messung durch das Kalibrierungsmodell:
- jede von dem Messsensor erfasste Messung (*X(t)*) die Eingabeschicht des neuronalen Netzes bildet;
- die Ausgabeschicht der Schätzung (*Z(t)*) mindestens einer Referenzmessung entspricht.

8. Verfahren nach einem der vorhergehenden Ansprüche, umfassend, zwischen den Schritten i) und ii), eine zeitliche Synchronisierung der Kalibrierungsmessungen in Bezug auf die Referenzmessungen.

9. Verfahren nach einem der vorhergehenden Ansprüche, wobei der Sensor mindestens umfasst:
- einen Bewegungssensor vom Typ Beschleunigungsmesser und/oder Gyrometer und/oder Magnetometer;
- und/oder einen Drucksensor;
- und/oder einen optischen Sensor;
- und/oder einen elektrischen Sensor;
- und/oder einen chemischen Sensor;
- und/oder einen Temperatursensor;
- und/oder einen physiologischen Sensor, der dazu ausgestaltet ist, ein physiologisches Merkmal des Benutzers zu messen.

10. Verfahren nach einem der vorhergehenden Ansprüche, bei dem der Zustand des Benutzers gewählt wird unter:
- einem Zustand, der eine körperliche Tätigkeit des Benutzers beschreibt;
- einem Stresszustand;
- einem Schlaf- oder Schläfrigkeitszustand;
- einem pathologischen Zustand;
- einem symptomatischen Zustand;
- einem Zustand, der einem Auftreten einer Gefahrensituation für den Benutzer entspricht.

11. Verfahren nach einem der vorhergehenden Ansprüche, bei dem der Benutzer ein lebender Mensch oder ein lebendes Tier ist.

12. Verfahren nach einem der vorhergehenden Ansprüche, bei dem die Auswerteapplikation von einem Mikroprozessor ausgeführt wird, der in die vernetzte Vorrichtung integriert ist, oder von einem Mikroprozessor, der von der vernetzten Vorrichtung entfernt ist und mit dieser über eine drahtgebundene oder drahtlose Verbindung verbunden ist.

13. Vernetzte Vorrichtung (1), die dazu bestimmt ist, von einem Benutzer getragen zu werden, und einen Messsensor (2) umfasst, wobei der Messsensor geeignet ist, zu verschiedenen Messzeitpunkten eine Messung (X(t)) einer physikalischen oder chemischen Grösse zu erfassen, die für eine Bewegung des Benutzers oder für ein physiologisches Merkmal des Benutzers repräsentativ ist, wobei der Messsensor durch Parameter des Sensors parametriert wird, wobei jeder Parameter des Sensors in einem Steuerregister einer Steuerschaltung des Sensors gespeichert wird, wobei die Vorrichtung dazu ausgestaltet ist, eine Auswerteapplikation (6) zu aktivieren, wobei die Auswerteapplikation dazu programmiert ist, ausgehend von den von dem Messsensor erfassten Messungen einen Zustand des Benutzers zu schätzen, wobei der Zustand des Benutzers unter mehreren vorbestimmten Zuständen gewählt wird, wobei die Vorrichtung **dadurch gekennzeichnet ist, dass** sie eine Zentraleinheit (5) umfasst, die programmiert ist, um :
- jede Messung der Messsequenz durch ein Kalibrierungsmodell zu verarbeiten, so dass ausgehend von jeder Messung eine Referenzmessung geschätzt wird;
- jede geschätzte Referenzmessung an die Auswerteapplikation (6) zu übertragen, wobei die Zentraleinheit (5) so konfiguriert ist, dass das Kalibrierungsmodell während einer Kalibrierungsphase erhalten wird, die verschiedene Kalibrierungszeitpunkte umfasst, wobei die Kalibrierungsphase die Schritte i) und ii) nach einem der vorhergehenden Ansprüche umfasst.
,

## Claims

1. A method for processing measurements, acquired by a measurement sensor (2), at various measurement times, the measurement sensor being:
- integrated into a connected device (1), worn/borne by a user, the connected device being configured to be connected to a wireless communication network;
- configured to acquire, at each measurement time, a measurement (*X*(*t*)) representative of a movement of the user or of a physiological characteristic of the user;
- connected to an electronic control circuit configured to command acquisition of measurements by the sensor, and/or pre-processing of measurements acquired by the sensor;
- parametrized by sensor parameters, each sensor parameter being stored in one control register of the electronic control circuit,
the method comprising:
- acquiring measurements by means of the measurement sensor at various measurement times, so as to form a sequence of measurements;
- transmitting data, established on the basis of the sequence of measurements, to an interpreting application (6), the interpreting application being programmed to estimate, on the basis of the transmitted data, a user state, the user state being selected from a plurality of predetermined states;
the method comprising, following acquisition of a sequence of measurements:
- processing each measurement (*X*(*t*)) of the sequence of measurements by means of a calibration model, so as to estimate, on the basis of each measurement, a reference measurement (*Z*(*t*));
- transmitting each estimated reference measurement to the interpreting application (6), the reference measurements then forming the data transmitted to the interpreting application;
the method being **characterized in that** the calibration model is established in the course of a calibrating phase, comprising various calibration times, the calibrating phase comprising:
- i) acquiring calibration measurements (*Xₖ*(*t*)) by means of the measurement sensor, at the various calibration times, and obtaining reference measurements (*Zₖ*(*t*)), at each calibration time, such that a reference measurement corresponds to each calibration measurement, at least one reference measurement being representative of a user state among the predetermined states, wherein the calibration measurements and the reference measurements measure the same chemical or physical quantity ;
- ii) on the basis of the reference measurements (*Zₖ*(*t*)) and of the calibration measurements (*Xₖ*(*t*)), defining a calibration model, the calibration model being configured to estimate reference measurements on the basis of measurements acquired by the measurement sensor.

2. The method as claimed in claim 1, wherein, in step i), the measurement sensor (2) is placed on a phantom, representative of at least one user state, the reference measurements being obtained from the phantom, configured to vary the physical quantity within a measurement range consistent with values that may be encountered under actual operating conditions.

3. The method as claimed in claim 2, wherein the phantom comprises a reference sensor, the reference sensor delivering a reference measurement at each calibration time.

4. The method as claimed in claim 1, wherein, in step i), the calibration sensor is placed on at least one test individual, the test individual also wearing/bearing a reference sensor, the reference sensor delivering a reference measurement at each calibration time.

5. The method as claimed in any one of the preceding claims, wherein the calibration model implements a supervised artificial-intelligence algorithm, the supervised artificial-intelligence algorithm being parametrized in the course of the calibrating phase.

6. The method as claimed in claim 5, wherein the supervised artificial-intelligence algorithm comprises a neural network.

7. The method as claimed in claim 6, wherein the neural network comprises a recurrent neural network, and preferably a bidirectional recurrent neural network, the neural network comprising an input layer and an output layer, such that in the course of processing of each measurement by the calibration model:
- each measurement (*X*(*t*)) acquired by the measurement sensor forms the input layer of the neural network;
- the output layer corresponds to the estimate (*Z*(*t*)) of at least one reference measurement.

8. The method as claimed in any one of the preceding claims comprising, between steps i) and ii), time synchronization of the calibration measurements with respect to the reference measurements.

9. The method as claimed in any one of the preceding claims, wherein the sensor comprises at least:
- a motion sensor, of the accelerometer and/or gyrometer and/or magnetometer type;
- and/or a pressure sensor;
- and/or an optical sensor;
- and/or an electrical sensor;
- and/or a chemical sensor;
- and/or a temperature sensor;
- and/or a physiological sensor, configured to determine a physiological characteristic of the user.

10. The method as claimed in any one of the preceding claims, wherein the user state is selected from:
- a state describing a physical activity of the user;
- a state of stress;
- a state of sleep or drowsiness;
- a pathological state;
- a symptomatic state;
- a state corresponding to occurrence of a situation putting the user at risk.

11. The method as claimed in any one of the preceding claims, wherein the user is a living human being or a living animal.

12. The method as claimed in any one of the preceding claims, wherein the interpreting application is implemented by a microprocessor integrated into the connected device, or by a microprocessor remote from the connected device and connected to the latter by a wired or wireless link.

13. A connected device (1), intended to be worn/borne by a user, comprising a measurement sensor (2), the measurement sensor being configured to acquire, at various measurement times, a measurement (X(t)) of a physical or chemical quantity, representative of a movement of the user or of a physiological characteristic of the user, the measurement sensor being parametrized by sensor parameters, each sensor parameter being stored in a control register of a control circuit of the sensor, the device being configured to activate an interpreting application (6), the interpreting application being programmed to estimate, on the basis of the measurements acquired by the measurement sensor, a user state, the user state being selected from a plurality of predetermined states, the device being **characterized in that** it comprises a central unit (5) programmed to :
- process each measurement by means of a calibration model, so as to estimate, on the basis of each measurement, a reference measurement;
- transmit each estimated reference measurement to the interpreting application
- the central unit being configured such that the calibration model is established in the course of a calibrating phase, comprising various calibration times, the calibrating phase comprising steps i) and ii) of any one of the preceding claims.
